# EUROPEAN PATENT SPECIFICATION

(11) **EP 3 377 006 B1**
(45) Date of publication and mention of the grant of the patent: **11.09.2019**
(21) Application number: 16798175.2
(22) Date of filing: 18.11.2016
(51) Int. Cl.: A61F 7/02, A61F 7/10, A61F 7/00

(54) **COOLING PAD FOR USE IN A NON-INVASIVE MEDICAL COOLING PROCESS FOR COOLING A BODY PART OF A PERSON**
KÜHLKISSEN ZUR BENÜTZUNG IN NICHT-INVASIVEM MEDIZINISCHEN KÜHLPROZESS ZUR KÜHLUNG EINES KÖRPERTEILS
COUSSIN DE REFROIDISSEMENT POUR L'USAGE DANS UN PROCÈDE DE REFROIDISSEMENT MÉDICAL NON-INVASIF POUR LE REFROIDISSEMENT D'UNE PARTIE DU CORPS

(30) Priority: 19.11.2015 SE 1551499
(43) Date of publication of application: 26.09.2018
(73) Proprietor: Braincool AB, 223 81 Lund (SE)
(72) Inventor: BERG, Jon, 247 54 Dalby (SE)
(74) Representative: Ström & Gulliksson AB
(86) International application number: PCT/EP2016/078164
(87) International publication number: WO 2017/085272

(56) References cited:
- WO-A1-2012/156761
- WO-A1-2013/007964
- WO-A2-01/62193
- WO-A2-2012/066339
- WO-A2-2015/120368
- US-A- 5 330 519
- US-A1- 2014 277 301

## Description

### TECHNICAL FIELD

The present invention relates to a cooling pad for medically cooling regions of the body of a person thereby reducing the body temperature in said regions.

### BACKGROUND

Cooling pads may be used in medical cooling processes for medically cooling various regions of the body of a person or patient in a non-invasive manner by placing the cooling pad onto or around the body part to be cooled. For example, a cooling pad may be employed to cool regions of the head or neck of a person who has suffered from stroke or to a concussion of the brain. Cooling pads may also be applied to cool the head of a person who is receiving chemo-therapy treatment in order to minimize the loss of hair which often is a result of the treatment.

A cooling pad typically consists of two superimposed sheets of flexible, fluid tight material that are sealed together along their edges. An inlet port is arranged in one end of the cooling pad for letting a flow of cooling fluid, e.g. liquid, into the pad and an outlet port is provided in another end allowing the flow of cooling liquid out of the pad.

A problem related to cooling pads is that the flow of cooling fluid may be unevenly distributed over the cooling pad and thereby resulting in a non-uniform and inefficient cooling effect.

This problem has been addressed in US4138743 which discloses a cooling pad to be applied on the head of a person. The cooling pad is divided into sections for cooling different portions of the head of the wearer of the pad, whereby each section comprises a plurality of flow passageways for distributing the cooling liquid over the respective section. However, due to the shape of the different sections of the cooling pad, the flow of liquid in the flow passageway may stagnate in certain regions of the cooling pad and result in a low cooling effect of the cooling pad.

US5086771 shows a further cooling pad having flow guiding means.

Thus, it is an object of the present disclosure to provide an improved cooling pad to be applied on portions of the body of person which solves or at least mitigates one or more of the problems of the prior-art. In particular, it is an object of the present disclosure to provide a cooling pad with increased cooling effect.

WO2012/156761 discloses a cooling pad with flow guides to deflect and diffuse flow. WO2012/066339 discloses a cooling pad with a W-shaped flow guide forming a fluid channel to provide flow. Further background prior art can be found in US2014/277301, US5330519, WO2013/007964, WO01/62193 and WO2015/120368.

### SUMMARY

According to a first aspect of the present disclosure at least one of these objects is achieved by a cooling pad for use in a non-invasive medical cooling process for cooling at least one portion of the body of a person by means of a cooling fluid flowing through the cooling pad in use. The cooling pad comprises:
- a bottom sheet and a top sheet of flexible and fluid tight material;
- an outer wall extending along the edges of the bottom and top sheets;
- an inlet opening for a flow of fluid and an outlet opening for a flow of fluid;
- at least one inner wall extending within the outer wall, wherein said inner wall is arranged such that a continuous flow channel for conveying fluid in a flow direction from the inlet opening to the outlet opening is formed between the outer wall, the inner wall and the bottom and top sheets, and;
- a plurality of flow guides to allow homogenous fluid flow across the flow channel, each flow guide for guiding a portion of a flow of fluid in the flow channel towards one of the inner wall or outer wall, wherein each flow guide is an elongate straight element and comprises a first end and a second end, wherein the flow guides are arranged on opposite sides of a centre axis extending through the flow channel, and wherein the flow guides are arranged in the flow channel such that the second end is directed towards one of the inner wall or the outer wall of the flow channel and such that the second end is arranged downstream of the first end with respect to the flow direction and such that the flow guide forms an acute angle with a centre axis extending through the flow channel.

The cooling pad of the present disclosure comprises a continuous flow channel which is formed between an outer wall extending along the edges of the cooling pad and an inner wall located within the outer wall. The flow channel thereby provides a unidirectional flow of fluid, i.e. a flow of fluid which essentially follows one single flow path from an inlet opening to an outlet opening of the cooling pad. Thereby are regions of stagnant fluid minimized in the cooling pad.

Typically, the flow of fluid in a flow channel is strongest in the centre of the channel and decreases towards the walls of the channel at which the flow may be stagnant. In the cooling pad according to the present disclosure the difference in flow rate across the flow channel is equalized by the provision of at least one flow guide which is extends oblique in downstream direction of the flow channel towards one of the walls. The flow guide thereby directs a portion of the strong flow of fluid in the centre of the flow channel towards the stagnant regions in the vicinity of the outer and inner walls. In summary, the provision of a unidirectional flow of fluid through the cooling pad and the equalizing effect provided by the flow guide on the flow of fluid across the flow channel results in that an even and effective cooling effect is achieved by the cooling pad according to the present disclosure. In addition thereto, the provision of the flow guide may further cause a turbulent fluid flow which also increases the cooling effect.

The cooling fluid may be any cooling medium suitable for conducting the medical cooling of the person or patient, e.g. a cooling liquid or a cooling gas.

Further alternatives of the present disclosure are disclosed in the appended claims and the following detailed description.

### BRIEF DESCRIPTION OF THE DRAWINGS

Fig. 1 is a schematic drawing of a cooling pad according to a preferred embodiment.
Fig. 2 is a schematic drawing identifying various sections of the cooling pad according to the preferred embodiment.
Fig. 3 is a schematic drawing showing the cooling pad according to the preferred embodiment applied onto the head and neck of a person.

### DETAILED DESCRIPTION

The cooling pad according to the present disclosure will now be described more fully hereinafter.

The cooling pad according to the present disclosure is hereinafter described with reference to a cooling pad configured for cooling portions of the head and neck of a person. However, it appreciated that the cooling pad according to the present disclosure alternatively may be configured for cooling other portions of the body of a person. For example, the cooling pad according to the present disclosure may be configured for cooling of the torso, or the back, or the legs, or the arms of a person.

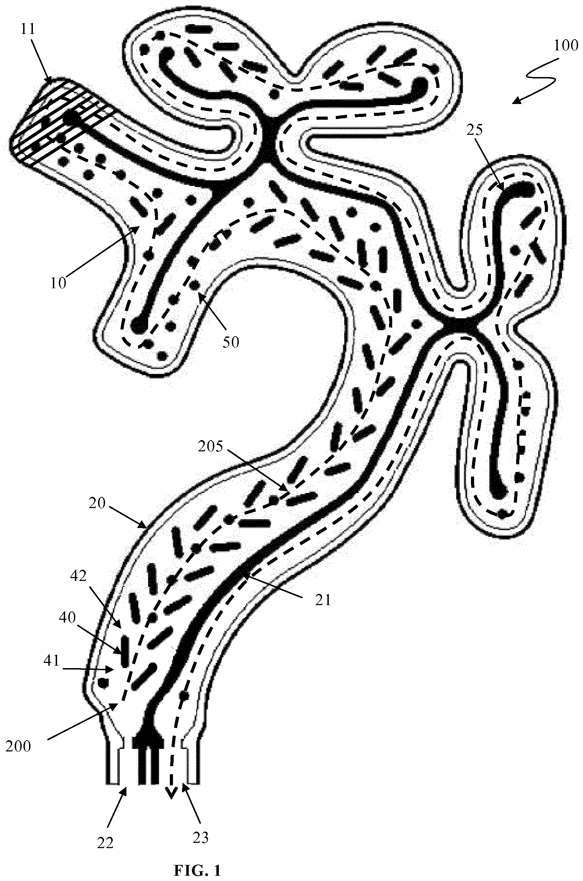

Fig. 1 shows a top view of a cooling pad 100 according to the present disclosure. It is appreciated that in operation, two cooling pads may be applied onto the head of a person, i.e. one cooling pad on either side of the head. Therefore, the cooling pad according to the present disclosure may also have the inverted shape of the cooling pad 100 shown in Fig. 1.

The cooling pad 100 comprises a bottom sheet 10 and a top sheet 11 (which only partially is shown in Fig. 1 in order not do obscure other features). The bottom and top sheets 10, 11 are manufactured of a flexible and fluid tight material such as silicon. The bottom and top sheets 10, 11 have the same shape and size and are superimposed such that their edges coincide. An outer wall 20 is arranged between the bottom and top sheets 10, 11. The outer wall 20 extends continuously along the edges of the bottom- and top sheets 10, 11 such that the outer wall 20 completely surrounds the periphery of the bottom- and the top sheets 10, 11. The outer wall 20 further comprises an opening which form an inlet and an outlet opening 22, 23 for fluid together with an inner wall 21, as will be described in the following.

Thus, the cooling pad 100 further comprises at least one inner wall 21. The inner wall 21 is arranged within the outer wall 20, and between the bottom- and top sheets 10, 11 The inner wall 21 divides the opening in the outer wall 20 in an inlet opening 22 for introducing a cooling fluid into the cooling pad and an outlet opening 23 for discharging fluid from the cooling pad. The inner wall 21 further extends within the cooling pad, at predetermined distances from the outer wall 20, such that a continuous flow channel 200 is achieved between the outer and inner walls 20, 21, from the inlet opening 22 to the outlet opening 23.

The flow direction from the inlet opening to the outlet opening is indicated by the dashed arrow 205. The dashed arrow 205 also marks the centre of the flow channel 200, thus it constitutes the centre axis 205 of the flow channel. The centre axis 205 extends in the longitudinal center of the flow channel through out the length of the flow channel and divides the flow channel in longitudinal direction into two opposite halves which have the same width.

When the cooling pad has a complex shape, such as shown in Fig. 1, the inner wall may comprise one or more branches 25 which extend into the various sections of the cooling pad. It is also possible that more than one inner wall is arranged in the cooling pad according to the present disclosure

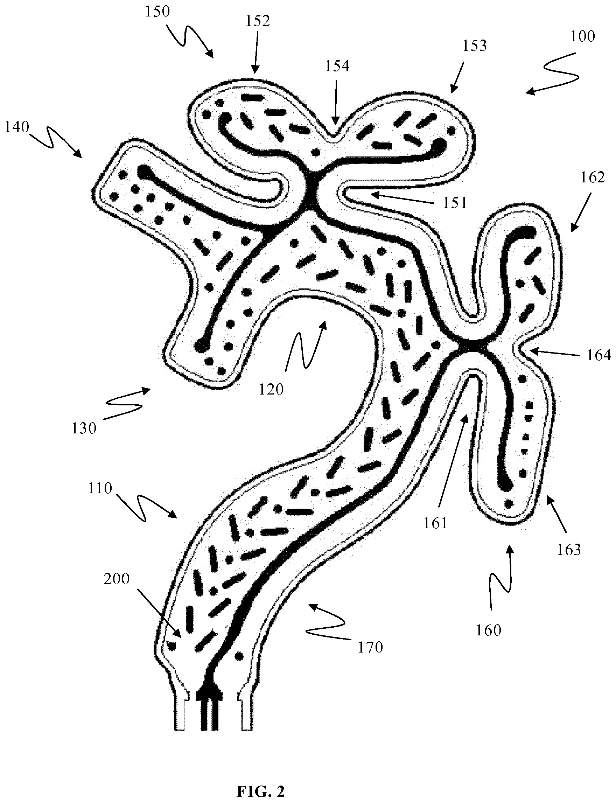

The cooling pad 100 of the embodiment shown in Fig. 1 is configured for cooling portions of the head and neck of a person. Following is, with reference to Fig. 2, a detailed description of sections of the cooling pad that are configured for cooling specific regions of the head or neck of a person. In the description below, the term "sections of the cooling pad" is used. However, it is appreciated that the term "sections of the cooling pad" also is equivalent to "sections of the flow channel". This is so since a section of the cooling pad which is configured for cooling a portion of the head or neck of a person is constituted by a portion of the flow channel.

Turning to Fig. 2, the cooling pad 100 may comprise a first section 110 which is configured to cool the cardial triangle of a person. This section is elongate and may extend from the inlet and outlet openings 22, 23 to a second section 120 which is configured for cooling the area behind the ear of a person. The second section 120 is of semi-circular shape, i.e. hook shaped, to fit around the ear of a person. The cooling pad 100 may further comprise a third section 130 which is joined to the second section 120. The third section 130 is configured to cool the temple of a person and is thereby of rectangular shape. A fourth section 140, configured to cool the forehead of a person may extend from the third section 130. Also the fourth section 140 is of rectangular shape. The cooling pad 100 may further comprise a fifth section 150 which is configured to cool the top of the head, i.e. the fontanel, of a person. The fifth section 150 comprises two elongate, rounded portions 152 and 153 which are joined by a waist 154 such that the fifth section 150 is heart-shaped. This shape allows the fifth section 150 to be seated without wrinkles onto the top of the head of a person. The fifth section 150 further comprises a stem portion 151 which joins the fifth section 150 to the rest of the cooling pad 100. The stem portion 151 is narrower than the two elongate, rounded portions 152, 153 and makes it possible to fold the two elongate, rounded portions 152 and 153 down onto the head of the user without causing wrinkles. The cooling pad 100 further comprises a sixth section 160 for cooling the back of the head of a user. Also this section needs to be folded onto the back of the head of a person to be placed on the rounded back head of the person without causing wrinkles. Therefore, also the sixth section 160 comprises two elongate, rounded portions 162 and 163 which are joined by a waist 164 and a stem portion 161 for attaching the sixth portion 160 to the rest of the cooling pad. The cooling pad 100 further comprises an outlet portion 170 which connects the flow channel 200 with the outlet opening 23. Thus, the flow channel 200 extends through all the sections 110, 120, 130, 140, 150, 160 and 170 in one single flow direction along the centre axis 205.

An advantage with the specific form of the cooling pad 100 according to the disclosure is that, when applied on to the head and neck of a person, it follows the form of the persons head very tightly and without wrinkles. This promotes heat transfer between the cooling pad and the head of the person.

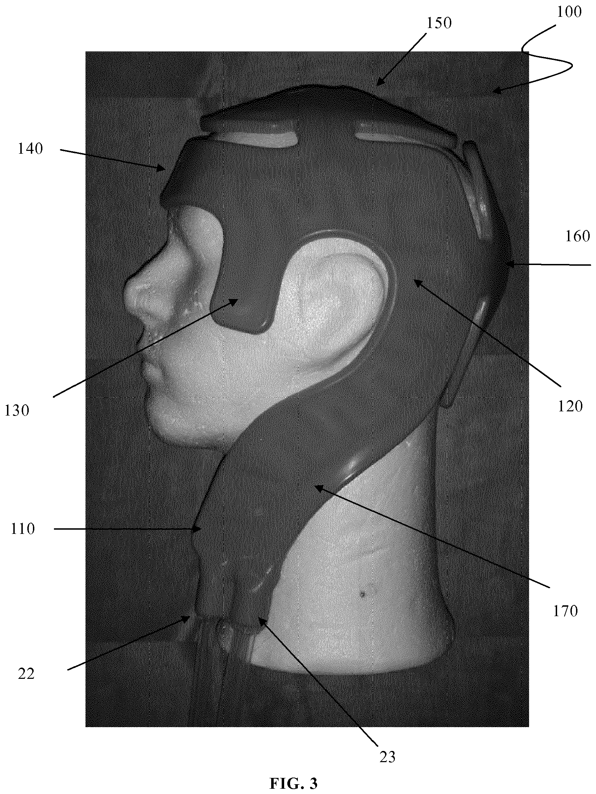

Fig. 3 shows a cooling pad 100 as described above applied to the head and neck of a person.

Returning to Fig. 1. According to a further aspect of the present disclosure, the cooling pad 100 comprises at least one flow guide 40 which is arranged in the flow channel 200 to direct, i.e. guide, a portion of a flow of fluid flowing through the flow channel 200 towards one of the outer wall 20 and the inner wall 21. By "one of the outer wall 20 and the inner wall 21" is meant the flow guide is arranged to direct the flow towards either the inner wall or towards the outer wall. The flow guide 40 is an elongate, straight element and comprises a first end 41 and a second end 42.

The flow guide 40 is oriented in the flow channel 200 such that its second end 42 is directed towards one of the outer wall 20 or the inner wall 21 and such that the second end 42 is positioned downstream first end 41. In other words, the flow guide 40 is oriented obliquely in respect to the flow of fluid in the flow channel 200 and will deflect the flow of fluid in downstream direction and towards the outer- or inner wall 20, 21.

The degree of oblique orientation of the flow guide 40 depends on the flow conditions in the flow channel and has to be determined for each particular case. However, the flow guide 40 forms an acute angle with the centre axis 205 of the flow channel. Preferably, the angle between the flow guide and centre axis is 30° - 60°.

The flow guide 40 may be arranged such that it extends across the centre axis 205 extending through the flow channel 200 (not shown in Fig.1).

However, as shown in Fig. 1 the flow guide 40 is preferably arranged such that it extends on one side of the centre axis 205. The flow guide 40 may thereby be arranged such that its first end 41 is positioned on the centre axis 205 in the flow channel 200 or at a predetermined distance from the centre axis 205 and such that its second end 42 is directed towards one of the outer wall 20 and the inner wall 21 of the flow channel 200. As described above, the second end 42 is positioned downstream the first end in respect of the flow direction.

The predetermined distance of the first end 41 of the flow guide 40 from the centre axis 205 is selected in dependency of the flow conditions that prevails in the flow channel 200.

The second end 42 of the flow guide is also positioned at a predetermined distance from the outer wall 20 or the inner wall 21. Also the position of the second end 42 of the flow guide 40 is determined in dependency of the flow conditions in the flow channel. It is preferred that the second end 42 of the flow guide is spaced apart from the outer- or inner wall in order to allow a flow of fluid adjacent to the outer- or inner wall.

The cooling pad may comprise a plurality of flow guides 40, arranged in the flow channel 200. By "plurality" of flow guides is meant 2 or more flow guides, such as 3, 4, 5, 10, 30, 50 or more. The plurality of flow guides 40 may be distributed in any suitable manner in the flow channel, for example one or both sides of the centre axis 205.

For example, the cooling pad may comprise , at least one pair of two flow guides 40, arranged side-by-side in the flow channel 200. The flow guides 40 are thereby arranged on opposite sides of the centre axis 205 of the flow channel and oriented such that the second end 42 of a first flow guide 40 is directed towards the inner wall 21 and the second end 42 of a second flow guide 40 is directed towards the outer wall 20. When a pair of two flow guides 40 are arranged side-by side in the flow channel, it is preferred that the each of the first ends 41 of the flow guides are at a predetermined distance from the centre axis 205 in order to allow a flow in the centre of the flow channel.

The cooling pad may comprise a plurality flow guides 40 that are arranged side-by-side on opposite sides of the centre axis 205 in the flow channel 200. It is thereby preferred that the flow guides 40 on one side of the centre axis 205 of the flow channel 200 are off-set axially along the centre axis 205 with respect to the flow guides 40 on the other side of the centre axis 205. This arrangement allows a portion of the strong flow in the center of the flow channel 200 to pass between the flow guides 40 while another portion of the flow is directed to the stagnant region close to the outer- and inner walls 10, 11 of the flow channel 200. The result is a homogenous fluid flow across the flow channel 200.

The flow guides 40 may be evenly distributed throughout the flow channel 200. However, the flow guides 40 are preferably arranged in selected regions of the flow channel 200 where a strong cooling effect is desired. For example, as shown in Fig. 2, a plurality of flow guides are arranged in the first section 110 for cooling the carotid artery and in the second section, 120 for cooling the area behind the ear of a person. These portions of the head and neck comprise large blood vessels and demand a strong cooling effect. Moreover, strong cooling of these portions of the body of a person results in less demand of cooling of downstream regions, such as the temple 130 and the forehead 140.

The cooling pad 200 according to the present disclosure may manufactured by forming the outer- and inner walls 20, 21 and the flow guides 40 integrally with one of the bottom- or top sheets 10, 11 and then attaching the other one of the bottom- and top sheet 10, 11 fluid tight on top of the outer- and inner walls 20, 21 and on top of the flow guides 40. Integral forming may be achieved by moulding techniques such as injection moulding or form pressing. Fluid tight attachment may be achieved by gluing.

The bottom and top sheets 10, 11 are typically made of silicon. The bottom sheet 10, which typically comprises the outer and inner walls 20, 21 and the flow guides 40 has a thickness of 1.5 mm. The top sheet 11, which in operation is in contact with regions of the head and neck of a person is thinner than the bottom sheet 10 to promote heat transfer and has thickness of 1.10 mm. The outer wall 20, the inner wall 21 and the flow guides 40 are also made of silicon and may have a height of 4 mm. The cooling fluid may typically be a mixture of 80% water and 20% ethylene glycol.

An additional effect of the flow guides 40 and the inner wall 21 is that the number of adherence points between the top and bottom sheets is increased. Thereby, the risk for so-called "ballooning" of the top sheet 11 is greatly reduced. Ballooning may be caused when the flow of fluid is through the flow channel 200 is obstructed within the cooling pad which in turn causes the static pressure to rise in the flow channel. Since the top sheet is made of very thin silicon it yields easily to the increased pressure in the flow channel and causes the cooling pad to swell up like a water balloon. The resulting volume increase causes the poor flow rate and low cooling effect.

Although a particular embodiment has been disclosed in detail this has been done for purpose of illustration only, and is not intended to be limiting. In particular it is contemplated that various substitutions, alterations and modifications may be made within the scope of the appended claims.

For example, to further minimize the risk of ballooning even further it is possible to arrange contact points 50 between the top and bottom sheets 10, 11. The contact points 50 are typically round elements having the same height as the flow guides 40 and the outer and inner walls 20, 21. Of-course, the bottom and top sheets 10, 11 are also fluid tight attached to the contact elements 50.

Preferably, the outlet opening 23 has larger diameter than the inlet opening 22 in order to avoid increase of the static pressure in the cooling pad. Preferably, the diameter of the outlet opening is twice the diameter of the inlet opening.

## Claims

1. A cooling pad (100) for use in a non-invasive medical cooling process to cool at least one portion of the body of a person by means of a cooling fluid flowing through said cooling pad in use, comprising:
- a bottom sheet (10) and a top sheet (11) of flexible and fluid-tight material;
- an outer wall (20) extending along the edges of the bottom and top sheets (10, 11);
- an inlet opening (22) for receiving a flow of fluid and an outlet opening (23) for exiting a flow of fluid;
- at least one inner wall (21) extending within the outer wall (20), wherein said inner wall (21) is arranged such that a continuous flow channel (200) for conveying fluid in a flow direction from the inlet opening (22) to the outlet opening (23) is formed between the outer wall (20), the inner wall (21) and the bottom and top sheets (10, 11), and;
- a plurality of flow guides (40), each flow guide (40) for guiding a portion of a flow of fluid in the flow channel (200) towards one of the inner wall (21) or outer wall (20), wherein each flow guide (40) comprises a first end (41) and a second end (42), wherein the flow guides (40) are arranged on opposite sides of a centre axis (205) extending through the flow channel (200) and wherein each flow guide (40) is arranged in the flow channel (200) such that the second end (42) is directed towards one of the inner wall (21) or the outer wall (20) of the flow channel (200) and such that the second end (42) is arranged downstream of the first end (41) with respect to the flow direction
**characterised in that** each flow guide (40) is an elongate straight element and each flow guide (40) forms an acute angle with the centre axis (205) extending through the flow channel (200) to allow a homogenous fluid flow across the flow channel.

2. The cooling pad (100) according to claim 1, wherein each flow guide (40) is arranged in the flow channel (200) such that the first end (41) is positioned at a predetermined distance from a centre axis (205) extending through the flow channel (200).

3. The cooling pad (100) according to claim 1 or 2, wherein each flow guide (40) is arranged in the flow channel (200) such that that the second end (42) is positioned at a predetermined distance from the outer wall (20) or the inner wall (21).

4. The cooling pad (100) according to any one of the claims 1 to 3, wherein each flow guide (40) is arranged on one side of a centre line (205) extending through the flow channel (200).

5. The cooling pad (100) according to any one of the claims 1 to 4, wherein the flow guides (40) on one side of a centre axis (205) extending through the flow channel (200) are offset by a predetermined distance along the centre axis (205).

6. The cooling pad (100) according to any one of the claims 1 to 5, wherein a plurality of flow guides (40) are arranged in at least one selected section of the flow channel (200) which is configured to provide increased cooling capacity.

7. The cooling pad (100) according to any one of claims 1 to 6, wherein the inlet opening (22) and the outlet opening (23) are arranged adjacent each other and are separated by the inner wall (21).

8. The cooling pad (100) according to any one of the claims 1 to 7 arranged for cooling selected portions of the head and/or the neck of a person in use, and further comprising a first section (110) configured for cooling the cardial triangle of a person; and/or a second section (120) configured for cooling an area behind the ear of a person; and/or a third section (130) configured for cooling the temple of a person; and/or a fourth section (140) configured for cooling the forehead of a person; and/or a fifth section (150) for cooling the top of the head of a person; and/or a sixth section (160) configured for cooling the back-head of a person.

9. The cooling pad (100) according to any one of the claims 1 to 8, wherein the outer wall (20), the inner wall (21) and said flow guides (40) are formed integral with one of the bottom sheet (10) and the top sheet (11) and that the other of the bottom sheet (10) and the top sheet (11) is fluid tight attached to the outer wall (20), the inner wall (21) and said flow guides (40).

10. The cooling pad (100) according to any one of the claims 1 to 9, comprising at least one contact element (50) attached to the top sheet and the bottom sheet, said contact element (50) having round shape and being of the same height as said flow guides (40) and said outer- and inner wall (20, 21).

11. The cooling pad (100) according to any one of the claims 1 to 10, wherein the diameter of the inlet opening (22) is smaller than that of the outlet opening (23).

12. The cooling pad (100) according to any one of the claims 1 to 11, wherein the inner wall (21) comprises one or more branches (25) extending into various sections of the cooling pad.

## Patentansprüche

1. Kühlkissen (100) zur Verwendung in einem nicht-invasiven medizinischen Kühlverfahren, um zumindest einen Teil des Körpers einer Person mittels eines bei Gebrauch durch das Kühlkissen fließenden Kühlfluids zu kühlen, umfassend
- eine untere Platte (10) und eine obere Platte (11) aus flexiblem und fluiddichtem Material,
- eine Außenwand (20), die sich entlang der Ränder der unteren und oberen Platte (10, 11) erstreckt,
- eine Einlassöffnung (22) zur Aufnahme eines Fluidflusses und eine Auslassöffnung (23) zur Abgabe eines Fluidflusses,
- mindestens eine Innenwand (21), die sich innerhalb der Außenwand (20) erstreckt, wobei die Innenwand (21) derart angeordnet ist, dass zwischen der Außenwand (20), der Innenwand (21) und der oberen und unteren Platte (10, 11) ein kontinuierlicher Durchflusskanal (200) zum Transport eines Fluids in Flussrichtung von der Einlassöffnung (22) zur Auslassöffnung (23) ausgebildet ist, und
- eine Vielzahl von Durchflussführungen (40), jede Durchflussführung zur Führung eines Teils eines Fluidflusses im Durchflusskanal (200) in Richtung der Innenwand (21) oder Außenwand (20), wobei jede Durchflussführung (40) ein erstes Ende (41) und ein zweites Ende (42) umfasst, wobei die Durchflussführungen (40) auf gegenüberliegenden Seiten einer sich durch den Durchflusskanal (200) erstreckenden Mittelachse (205) angeordnet sind und wobei jede Durchflussführung (40) derart im Durchflusskanal (200) angeordnet ist, dass das zweite Ende (42) in Richtung der Innenwand (21) oder Außenwand (20) des Durchflusskanals (200) gerichtet ist und dass das zweite Ende (42) stromabwärts des ersten Endes (41) in Bezug auf die Durchflussrichtung angeordnet ist,
**dadurch gekennzeichnet, dass** jede Durchflussführung (40) ein längliches gerades Element ist und jede Durchflussführung (40) einen spitzen Winkel mit der sich durch den Durchflusskanal (200) erstreckenden Mittelachse (205) bildet, um einen homogenen Fluiddurchfluss durch den Durchflusskanal zu ermöglichen.

2. Kühlkissen (100) nach Anspruch 1, **dadurch gekennzeichnet, dass** jede Durchflussführung (40) derart im Durchflusskanal (200) angeordnet ist, dass das erste Ende (41) in einem vorgegebenen Abstand von einer sich durch den Durchflusskanal (200) erstreckenden Mittelachse (205) positioniert ist.

3. Kühlkissen (100) nach Anspruch 1 oder 2, **dadurch gekennzeichnet, dass** jede Durchflussführung (40) derart im Durchflusskanal (200) angeordnet ist, dass das zweite Ende (42) in einem vorgegebenen Abstand von der Außenwand (20) oder der Innenwand (21) positioniert ist.

4. Kühlkissen (100) nach einem der Ansprüche 1 bis 3, **dadurch gekennzeichnet, dass** die Durchflussführung (40) auf einer Seite einer sich durch den Durchflusskanal (200) erstreckenden Mittelachse (205) angeordnet ist.

5. Kühlkissen (100) nach einem der Ansprüche 1 bis 4, **dadurch gekennzeichnet, dass** die Durchflussführungen (40) auf einer Seite einer sich durch den Durchflusskanal (200) erstreckenden Mittelachse (205) um einen vorgegebenen Abstand entlang der Mittelachse (205) versetzt sind.

6. Kühlkissen (100) nach einem der Ansprüche 1 bis 5, **dadurch gekennzeichnet, dass** eine Vielzahl von Durchflussführungen (40) in mindestens einem Abschnitt des Durchflusskanals (200) angeordnet sind, welcher zur Bereitstellung einer erhöhten Kühlleistung vorgesehen ist.

7. Kühlkissen (100) nach einem der Ansprüche 1 bis 6, **dadurch gekennzeichnet, dass** die Einlassöffnung (22) und die Auslassöffnung (23) nebeneinander angeordnet und durch die Innenwand (21) getrennt sind.

8. Kühlkissen (100) nach einem der Ansprüche 1 bis 9, das zum Kühlen ausgewählter Abschnitte des Kopfes und/oder des Nackens einer verwendenden Person ausgebildet ist und ferner einen ersten Abschnitt (110), welcher zum Kühlen des Herzdreiecks einer Person vorgesehen ist, und/oder einen zweiten Abschnitt (120), welcher zum Kühlen eines Bereichs hinter dem Ohr einer Person vorgesehen ist, und/oder einen dritten Abschnitt (130), welcher zum Kühlen der Schläfe einer Person vorgesehen ist, und/oder einen vierter Abschnitt (140), welcher zum Kühlen der Stirn einer Person vorgesehen ist, und/oder einen fünften Abschnitt (150), welcher zum Kühlen des Oberkopfes einer Person vorgesehen ist, und/oder einen sechsten Abschnitt (160), welcher zum Kühlen des Hinterkopfes einer Person vorgesehen ist, umfasst.

9. Kühlkissen (100) nach einem der Ansprüche 1 bis 8, **dadurch gekennzeichnet, dass** die Außenwand (20), die Innenwand (21) und die Durchflussführungen (40) entweder mit der unteren Platte (10) oder der oberen Platte (11) integral ausgebildet sind und dass die jeweils andere untere Platte (10) oder obere Platte (11) an der Außenwand (20), der Innenwand (21) und den Durchflussführungen (40) fluiddicht befestigt ist.

10. Kühlkissen (100) nach einem der Ansprüche 1 bis 9, umfassend mindestens ein an der oberen Platte und der unteren Platte befestigtes Kontaktelement (50), wobei das Kontaktelement (50) eine runde Form und die gleiche Höhe wie die mindestens eine Durchflussführung (40) und die Außen- und Innenwand (20, 21) aufweist.

11. Kühlkissen (100) nach einem der Ansprüche 1 bis 10, **dadurch gekennzeichnet, dass** der Durchmesser der Einlassöffnung (22) kleiner ist als der Durchmesser der Auslassöffnung (23).

12. Kühlkissen (100) nach einem der Ansprüche 1 bis 11, **dadurch gekennzeichnet, dass** die Innenwand (21) eine oder mehrere Abzweigungen (25) umfasst, die sich in verschiedene Abschnitte des Kühlkissens erstrecken.

## Revendications

1. Compresse de refroidissement (100) à utiliser dans un processus de refroidissement médical non invasif pour refroidir au moins une portion du corps d'une personne au moyen d'un fluide de refroidissement s'écoulant à travers ladite compresse de refroidissement en utilisation, comprenant :
- une feuille basse (10) et une feuille haute (11) en matériau flexible et étanche aux fluides ;
- une paroi externe (20) s'étendant le long des bords des feuilles basse et haute (10, 11) ;
- une ouverture d'admission (22) pour recevoir un écoulement de fluide et une ouverture d'évacuation (23) pour faire sortir un écoulement de fluide ;
- au moins une paroi interne (21) s'étendant au sein de la paroi externe (20), dans lequel ladite paroi interne (21) est agencée de telle sorte qu'un canal d'écoulement continu (200) destiné à acheminer un fluide dans une direction d'écoulement allant de l'ouverture d'admission (22) à l'ouverture d'évacuation (23) est formé entre la paroi externe (20), la paroi interne (21) et les feuilles basse et haute (10, 11), et ;
- une pluralité de guides d'écoulement (40), chaque guide d'écoulement (40) étant destiné à guider une portion d'un écoulement de fluide dans le canal d'écoulement (200) vers l'une de la paroi interne (21) ou de la paroi externe (20), dans lequel chaque guide d'écoulement (40) comprend une première extrémité (41) et une seconde extrémité (42), dans lequel les guides d'écoulement (40) sont agencés sur des côtés opposés d'un axe central (205) s'étendant au travers du canal d'écoulement (200) et dans lequel chaque guide d'écoulement (40) est agencé dans le canal d'écoulement (200) de telle sorte que la seconde extrémité (42) est dirigée vers l'une de la paroi interne (21) ou de la paroi externe (20) du canal d'écoulement (200) et de telle sorte que la seconde extrémité (42) est agencée en aval de la première extrémité (41) par rapport à la direction d'écoulement
**caractérisé en ce que** chaque guide d'écoulement (40) est un élément droit allongé et chaque guide d'écoulement (40) forme un angle aigu avec l'axe central (205) s'étendant au travers du canal d'écoulement (200) pour permettre un écoulement de fluide homogène à travers le canal d'écoulement.

2. Compresse de refroidissement (100) selon la revendication 1, dans laquelle chaque guide d'écoulement (40) est agencé dans le canal d'écoulement (200) de telle sorte que la première extrémité (41) est positionnée à une distance prédéterminée d'un axe central (205) s'étendant au travers du canal d'écoulement (200).

3. Compresse de refroidissement (100) selon la revendication 1 ou 2, dans laquelle chaque guide d'écoulement (40) est agencé dans le canal d'écoulement (200) de telle sorte que la seconde extrémité (42) est positionnée à une distance prédéterminée de la paroi externe (20) ou de la paroi interne (21).

4. Compresse de refroidissement (100) selon l'une quelconque des revendications 1 à 3, dans laquelle chaque guide d'écoulement (40) est agencé sur un côté d'une ligne centrale (205) s'étendant au travers du canal d'écoulement (200).

5. Compresse de refroidissement (100) selon l'une quelconque des revendications 1 à 4, dans laquelle les guides d'écoulement (40) sur un côté d'un axe central (205) s'étendant à travers le canal d'écoulement (200) sont décalés d'une distance prédéterminée le long de l'axe central (205).

6. Compresse de refroidissement (100) selon l'une quelconque des revendications 1 à 5, dans laquelle une pluralité de guides d'écoulement (40) sont agencés dans au moins une section sélectionnée du canal d'écoulement (200) qui est configuré pour conférer une capacité de refroidissement accrue.

7. Compresse de refroidissement (100) selon l'une quelconque des revendications 1 à 6, dans laquelle l'ouverture d'admission (22) et l'ouverture d'évacuation (23) sont agencées adjacentes l'une à l'autre et sont séparées par la paroi interne (21).

8. Compresse de refroidissement (100) selon l'une quelconque des revendications 1 à 7, agencée pour refroidir des portions sélectionnées de la tête et/ou du cou d'une personne en utilisation, et comprenant en outre une première section (110) configurée pour refroidir le triangle cardial d'une personne ; et/ou une deuxième section (120) configurée pour refroidir une zone derrière l'oreille d'une personne ; et/ou une troisième section (130) configurée pour refroidir la tempe d'une personne ; et/ou une quatrième section (140) configurée pour refroidir le front d'une personne ; et/ou une cinquième section (150) pour refroidir le haut de la tête d'une personne ; et/ou une sixième section (160) configurée pour refroidir l'arrière de la tête d'une personne.

9. Compresse de refroidissement (100) selon l'une quelconque des revendications 1 à 8, dans laquelle la paroi externe (20), la paroi interne (21) et lesdits guides d'écoulement (40) sont formés solidaires de l'une de la feuille basse (10) et de la feuille haute (11) et en ce que l'autre de la feuille basse (10) et de la feuille haute (11) est fixée de manière étanche aux fluides à la paroi externe (20), à la paroi interne (21) et auxdits guides d'écoulement (40).

10. Compresse de refroidissement (100) selon l'une quelconque des revendications 1 à 9, comprenant au moins un élément de contact (50) fixé à la feuille haute et à la feuille basse, ledit élément de contact (50) ayant une forme ronde et étant de même hauteur que lesdits guides d'écoulement (40) et ladite paroi externe et interne (20, 21).

11. Compresse de refroidissement (100) selon l'une quelconque des revendications 1 à 10, dans laquelle le diamètre de l'ouverture d'admission (22) est plus petit que celui de l'ouverture d'évacuation (23).

12. Compresse de refroidissement (100) selon l'une quelconque des revendications 1 à 11, dans laquelle la paroi interne (21) comprend une ou plusieurs branches (25) s'étendant dans diverses sections de la compresse de refroidissement.
